# EUROPEAN PATENT APPLICATION

(11) **EP 2 873 736 A1**
(43) Date of publication of application: **20.05.2015**
(21) Application number: 13193270.9
(22) Date of filing: 18.11.2013
(51) Int. Cl.: C12P 35/00

(54) **Chemoenzymatic synthesis of the cephalosporine intermediate 7-amino-3-alkenylcephem-4-carboxylic acid**

(71) Applicant: Sandoz AG, 4056 Basel (CH)
(72) Inventor: The designation of the inventor has not yet been filed
(74) Representative: Wibbelmann, Jobst

(57) **Abstract**

The invention relates to processes for the preparation of 7-amino-3-alkenylcephem-4-carboxylic acid of Formula 6 and the compounds of Formula 2 and 5 by using the enzymes p-nitrobenzyl esterase and penicillin G amidase. The processes either start from the compound of Formula 1 or the compound of Formula 5.

SEQUENCE LISTING

<110>  Sandoz AG

<120>  Chemoenzymatic synthesis of the cephalosporine intermediate
       7-amino-3-alkenylcephem-4-carboxylic acid

<130>  EPA-121 866

<160>  1

<170>  PatentIn version 3.5

<210>  1
<211>  489
<212>  PRT
<213>  Bacillus subtilis

<400>  1

Met Thr His Gln Ile Val Thr Thr Gln Tyr Gly Lys Val Lys Gly Thr
1               5                   10                  15

Thr Glu Asn Gly Val His Lys Trp Lys Gly Ile Pro Tyr Ala Lys Pro
            20                  25                  30

Pro Val Gly Gln Trp Arg Phe Lys Ala Pro Glu Pro Pro Glu Val Trp
        35                  40                  45

Glu Asp Val Leu Asp Ala Thr Ala Tyr Gly Pro Ile Cys Pro Gln Pro
    50                  55                  60

Ser Asp Leu Leu Ser Leu Ser Tyr Thr Glu Leu Pro Arg Gln Ser Glu
65                  70                  75                  80

Asp Cys Leu Tyr Val Asn Val Phe Ala Pro Asp Thr Pro Ser Gln Asn
                85                  90                  95

Leu Pro Val Met Val Trp Ile His Gly Gly Ala Phe Tyr Leu Gly Ala
            100                 105                 110

Gly Ser Glu Pro Leu Tyr Asp Gly Ser Lys Leu Ala Ala Gln Gly Glu
        115                 120                 125

Val Ile Val Val Thr Leu Asn Tyr Arg Leu Gly Pro Phe Gly Phe Leu
    130                 135                 140

His Leu Ser Ser Phe Asp Glu Ala Tyr Ser Asp Asn Leu Gly Leu Leu
145                 150                 155                 160

Asp Gln Ala Ala Ala Leu Lys Trp Val Arg Glu Asn Ile Ser Ala Phe
                165                 170                 175

Gly Gly Asp Pro Asp Asn Val Thr Val Phe Gly Glu Ser Ala Gly Gly
            180                 185                 190

Met Ser Ile Ala Ala Leu Leu Ala Met Pro Ala Ala Lys Gly Leu Phe
        195                 200                 205

Gln Lys Ala Ile Met Glu Ser Gly Ala Ser Arg Thr Met Thr Lys Glu
    210                 215                 220

Gln Ala Ala Ser Thr Ala Ala Ala Phe Leu Gln Val Leu Gly Ile Asn
225                 230                 235                 240

Glu Ser Gln Leu Asp Arg Leu His Thr Val Ala Ala Glu Asp Leu Leu
                245                 250                 255

Lys Ala Ala Asp Gln Leu Arg Ile Ala Glu Lys Glu Asn Ile Phe Gln
            260                 265                 270

Leu Phe Phe Gln Pro Ala Leu Asp Pro Lys Thr Leu Pro Glu Glu Pro
        275                 280                 285

Glu Lys Ser Ile Ala Glu Gly Ala Ala Ser Gly Ile Pro Leu Leu Ile
    290                 295                 300

Gly Thr Thr Arg Asp Glu Gly Tyr Leu Phe Phe Thr Pro Asp Ser Asp
305                 310                 315                 320

Val His Ser Gln Glu Thr Leu Asp Ala Ala Leu Glu Tyr Leu Leu Gly
                325                 330                 335

Lys Pro Leu Ala Glu Lys Ala Ala Asp Leu Tyr Pro Arg Ser Leu Glu
            340                 345                 350

Ser Gln Ile His Met Met Thr Asp Leu Leu Phe Trp Arg Pro Ala Val
        355                 360                 365

Ala Tyr Ala Ser Ala Gln Ser His Tyr Ala Pro Val Trp Met Tyr Arg
    370                 375                 380

Phe Asp Trp His Pro Glu Lys Pro Pro Tyr Asn Lys Ala Phe His Ala
385                 390                 395                 400

Leu Glu Leu Pro Phe Val Phe Gly Asn Leu Asp Gly Leu Glu Arg Met
                405                 410                 415

Ala Lys Ala Glu Ile Thr Asp Glu Val Lys Gln Leu Ser His Thr Ile
            420                 425                 430

Gln Ser Ala Trp Ile Thr Phe Ala Lys Thr Gly Asn Pro Ser Thr Glu
        435                 440                 445

Ala Val Asn Trp Pro Ala Tyr His Glu Glu Thr Arg Glu Thr Val Ile
    450                 455                 460

Leu Asp Ser Glu Ile Thr Ile Glu Asn Asp Pro Glu Ser Glu Lys Arg
465                 470                 475                 480

Gln Lys Leu Phe Pro Ser Lys Gly Glu
                485

## Description

The invention relates to processes for the preparation of 7-amino-3-alkenylcephem-4-carboxylic acid of Formula 6, preferably 7-amino-3-vinylcephem-4-carboxylic acid of Formula 6' (VICA), and intermediate products of Formula 2, preferably of Formula 2' (G-VICA), and of Formula 5, preferably of Formula 5' (*p*MB-VICA) by using the enzymes *p*-nitrobenzyl esterase and penicillin G amidase. The processes either start from the compound of Formula 1, preferably of Formula 1'(G-Vinyl-E) or the compound of Formula 5/5'.

### Background prior art

The compound *p*-methoxybenzyl-7-phenylacetamido-3-vinylcephem-4-carboxylate (also named GVinylE) of Formula 1' 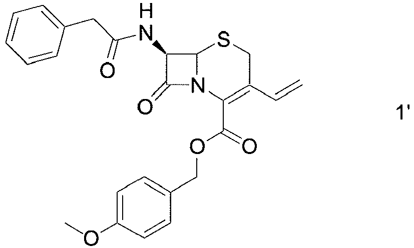 can be used for preparing the cephalosporine intermediate 7-amino-3-vinylcephem-4-carboxylic acid (also named VICA) of Formula 6'

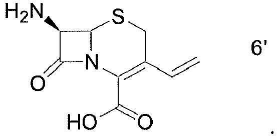

US 6,417,351 B1 (see e.g. Example 1) discloses the preparation of the compound of Formula 1' by applying a Wittig reaction. It is known that the protection group in 4-position of a cephem ring like in the compound of Formula 1' and the protection group at the amino group can be removed by using chemical reagents (see e.g. Hu et al., J. China Pharm. Univ., 1993, 24(4):246-247, reaction scheme).

For example, US 6,420,554 discloses the deprotection of a cephem ring in 4-position by using phenols and cresols.

Instead of using chemical reagents for deprotecting the amino group in the intermediate compound of Formula 2'

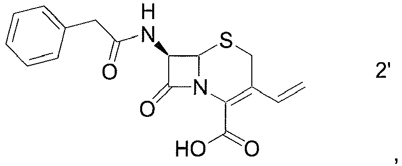

Chao et al. (Organic Process Research & Development 2009, 13, 924-927) describes an enzymolysis with penicillin G amidase (PenG). An enzymatic hydrolysis of the compounds of Formula 1' and 5', however, is not described in the art.

The use of enzymes other than penicillin G amidase is for example known in the art for the removal of a *p*-nitrobenzyl ester protection group at the amino group of a cephem ring. As disclosed in EP 0 549 264 A1, *p*-nitrobenzyl esterase (also referred to as "PNB esterase" or pNBE) can be derived from a microorganism of the genus Bacillus. The enzyme is a monomer having a molecular weight of about 54,000 daltons. This PNB esterase is described to catalyze the deesterification of PNB-esters of cephalosporin and 1-carbacephalosporin compounds to the free acid form.

US 3,972,774 describes the use of *p*-nitrobenzyl esterases for the deesterification of cephalosporin p-nitrobenzyl esters.

US 5,945,325 B1 discloses the preparation of *p*-nitrobenzyl esterases by modification of *p-*nitrobenzyl esterases from *Bacillus subtilis.*

In view of the above processes there is a need to prepare VICA and intermediates thereof by using lesser hazardous chemicals while still providing a high product yield.

### Summary of the invention

The invention relates to a process for the preparation of 7-amino-3-alkenylcephem-4-carboxylic acid of Formula 6 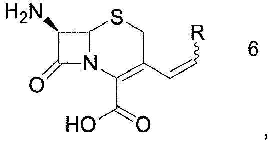 wherein, R = H or methyl, preferably R = H, wherein if R = H, the compound is 7-amino-3-vinylcephem-4-carboxylic acid of Formula 6' 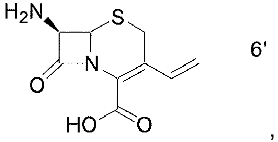 comprising steps (i) to (iii):
(i) providing a compound of Formula 1 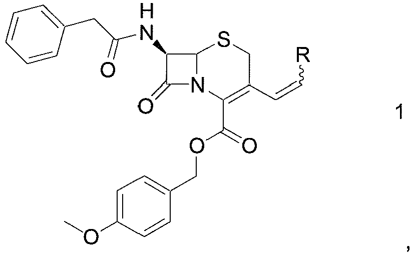 wherein R is as defined above, preferably R = H, wherein if R = H, the compound is defined by Formula 1' 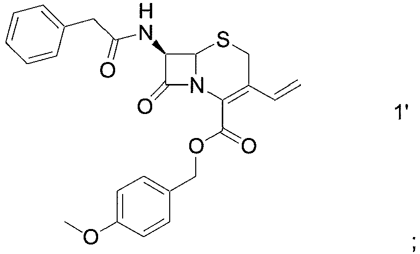
(ii) performing one of alternatives (A), (B) and (C):
   (A)
      (a) subjecting the compound of Formula 1, preferably of Formula 1', of step (i) to an enzymatic ester hydrolysis by using *p*-nitrobenzyl esterase in order to provide the compound of Formula 2 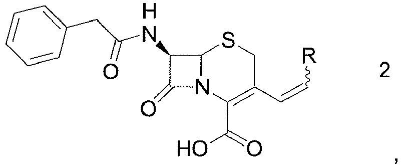 wherein R is as defined above, preferably the compound of Formula 2' 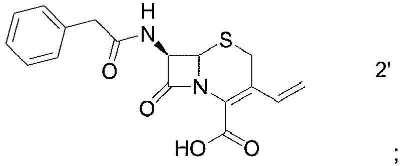 and the compound of Formula 3 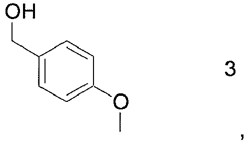 and
      (b) subjecting the compound of Formula 2, preferably of Formula 2', of step (a) to an amide bond cleavage by using pencillin G amidase in order to provide the compound of Formula 6, preferably of Formula 6', and the compound of Formula 4 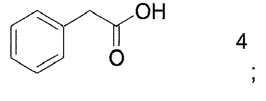
   (B)
      (c) subjecting the compound of Formula 1, preferably of Formula 1', of step (i) to an amide bond cleavage by using pencillin G amidase in order to provide the compound of Formula 5 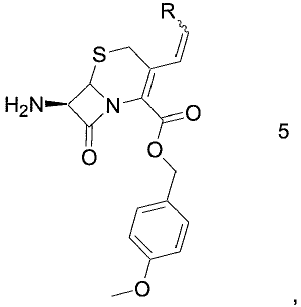 wherein R is as defined above, preferably the compound of Formula 5' 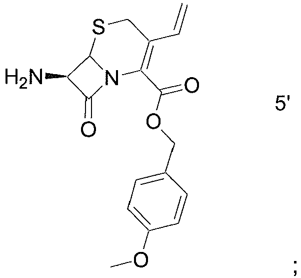 and the compound of Formula 4, and
      (d) subjecting the compound of Formula 5, preferably of Formula 5', of step (c) to an enzymatic ester hydrolysis with *p*-nitrobenzyl esterase in order to provide the compound of Formula 6, preferably of Formula 6', and the compound of Formula 3; and
   (C)
      performing sequences A and B as a one-pot reaction by contacting the compound of Formula 1, preferably of Formula 1', of step (i) with p-nitrobenzyl esterase and pencillin G amidase; and
(iii) obtaining the compound of Formula 6, preferably, of Formula 6'.

The invention also refers to a process for the preparation of the compound of Formula 2 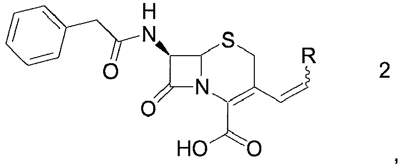 wherein R = H or methyl, preferably R = H, wherein if R = H, the compound is defined by Formula 2' 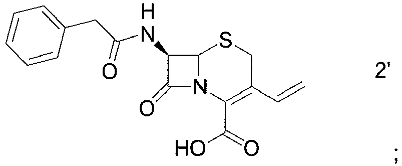 comprising steps (i) and (ii)(A)(a) as defined above and more detailed below, and a step of obtaining the compound of Formula 2.

The invention also refers to a process for the preparation of 7-amino-3-alkenylcephem-4-carboxylic acid of Formula 6, preferably of Formula 6', comprising steps (I) to (III):
(I) providing a compound of Formula 5, preferably of Formula 5';
(II) subjecting the compound of Formula 5, preferably of Formula 5', to a process as defined in step (ii)(B)(d) above and more detailed below; and
(III) obtaining the compound of Formula 6, preferably of Formula 6'.

The invention also refers to a process for the preparation of the compound of Formula 5, preferably of Formula 5', comprising step (i) and (ii)B(c) as defined above and more detailed below and an additional step of obtaining the compound of Formula 5/5'.

The invention also refers to the use of a *p*-nitrobenzyl esterase as defined herein for the preparation of the compound of Formula 2, preferably of Formula 2', or the compound of Formula 6, preferably the compound of Formula 6'.

The invention also refers to the use of *p*-nitrobenzyl esterase as defined herein and pencillin G amidase as defined herein for the preparation of the compound of Formula 6, preferably the compound of Formula 6'.

### List of figures

- Figure 1:: Figure 1 shows a general reaction scheme for the hydrolysis reactions of the present invention.
- Figure 2:: Figure 2 shows the ester hydrolysis of the compound of Formula 1' by mutant 5-6C8 of *p*-nitrobenzyl esterase from *Bacillus subtilis.*
- Figure 3:: Figure 3 shows the conversion of Compound 5' to Compound 6' by using mutant 5-6C8 of *p*-nitrobenzyl esterase from *Bacillus subtilis.*
- Figure 4:: Figure 4 shows the conversion of Compound 1' to Compound 2' by using the mutant 5-6C8 of *p*-nitrobenzyl esterase from *Bacillus subtilis.*

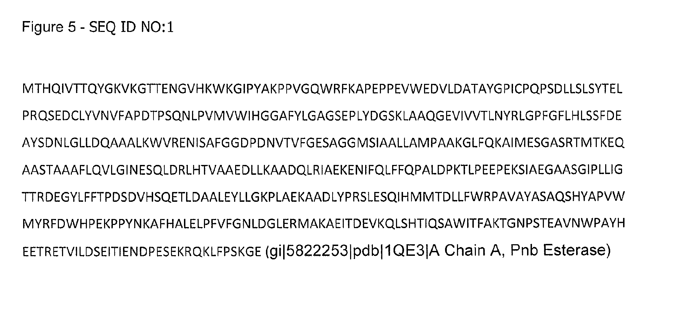

- Figure 5:: Figure 5 shows SEQ ID NO: 1 of *p*-nitrobenzyl esterase.

### Detailed Description

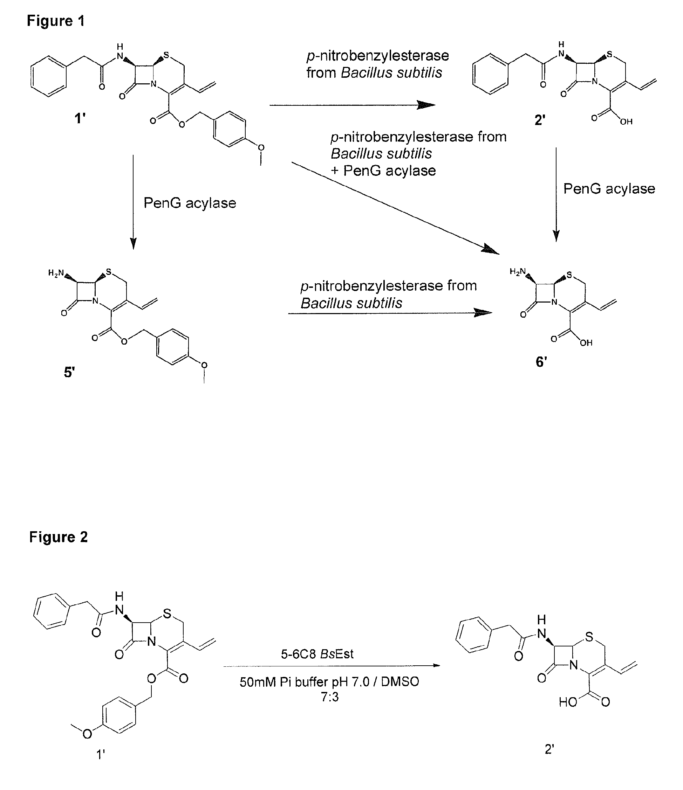

It has unexpectedly been found in the context of the present invention that the compound of Formula 6' (VICA), can be prepared from the compound of Formula 1' (G-Vinyl-E) by using enzymes (see general Scheme in Figure 1). In particular, it has unexpectedly been found that *p-*nitrobenzyl esterase from *Bacillus subtilis* is suitable for effective deprotection of the *p-*methoxybenzyl ester at position 4 of the cephem ring of the compounds of Formula 1' and 5' (pMB-VICA) as demonstrated herein. The inventors expect that due to similar chemical properties, the compound of Formula 1 where R = methyl, will react in the same manner as the compound of Formula 1'. The compound of Formula 6 with R = methyl is called PACA (7-amino-3-(1-propen-1-yl)-cephem-4-carboxylic acid)

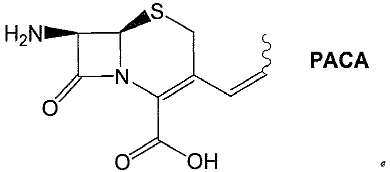

Particular problems related with the use of the compound of Formula 1 as starting material are the high stability of the protection group, the low solubility of G-Vinyl-E in water which requires the use of organic solvents as well as the low stability of cephem rings in general. In the context of the present invention, a variety of enzymes showed not suitable for deprotection of said *p-*methoxybenzyl ester. Since G-Vinyl-E has conjugated double bonds (vinyl-Group, ring and carboxylic group) which stabilize the ester bond, this p-methoxybenzyl ester can only be cleaved by strong acids like CF₃COOH or phenol/m-cresol as known in the art. It was therefore unexpected that the stable *p*-methoxybenzyl ester could be cleaved by applying the method of the present invention.

Although Chao et al. (Organic Process Research & Development 2009, 13, 924-927) describe an enzymolysis of the compound of Formula 2' (G-VICA), it was unexpected that PenG removes phenylacetic acid from G-Vinyl-E (Compound 1'), since the solubility of G-Vinyl-E is lower than that of G-VICA. The product VICA is quite soluble in water which pulls the equilibrium towards the VICA side when starting from G-VICA. This cannot be assumed in the case of G-Vinyl-E cleavage.

It was therefore unexpected that VICA and intermediates thereof can be prepared by using lesser hazardous chemicals as used in prior art processes, i.e. by using enzymes in aqueous solutions having a low organic solvent content, while still providing a high product yield.

It has also been found that a reduction of the amount of organic solvents such as DMSO in the reaction mixtures has a positive effect on the reaction.

The term "compound of Formula" and "compound" are interchangeably used in the context with the numbering used herein, i.e. "compound of Formula 1" has the same meaning as "Compound 1 ".

The invention thus refers to a process for the preparation of 7-amino-3-alkenylcephem-4-carboxylic acid of Formula 6 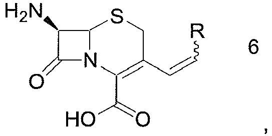 wherein, R = H or methyl, preferably R = H, wherein if R = H, the compound is 7-amino-3-vinylcephem-4-carboxylic acid of Formula 6' 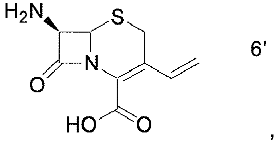 comprising, preferably consisting of, steps (i) to (iii):
(i) providing a compound of Formula 1 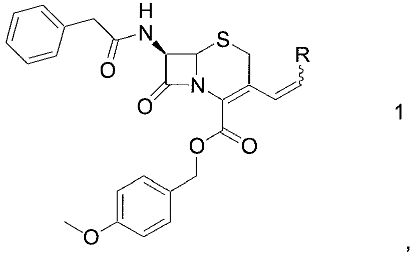 wherein R is as defined above, preferably R = H, wherein if R = H, the compound is defined by Formula 1' 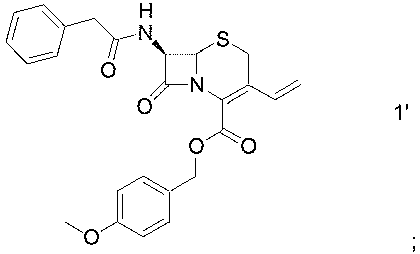
(ii) performing one of alternatives (A), (B) and (C):
   (A) (synthesis sequence: Compound 1 -> Compound 2 -> Compound 6)
      (a) subjecting the compound of Formula 1/1' of step (i) to an enzymatic ester hydrolysis by using *p*-nitrobenzyl esterase in order to provide the compound of Formula 2 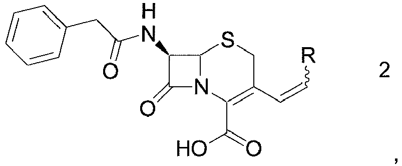 preferably the compound of Formula 2' 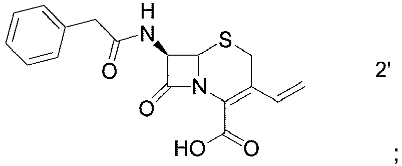 and the compound of Formula 3 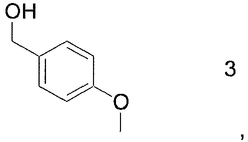 optionally isolating the compound of Formula 2/2' from the reaction mixture, and
      (b) subjecting the compound of Formula 2/2' of step (a) to an amide bond cleavage by using pencillin G amidase in order to provide the compound of Formula 6/6' and the compound of Formula 4 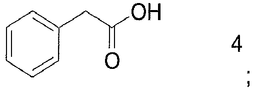
   (B) (synthesis sequence: Compound 1 -> Compound 5 -> Compound 6)
      (c) subjecting the compound of Formula 1/1' of step (i) to an amide bond cleavage by using pencillin G amidase in order to provide the compound of Formula 5 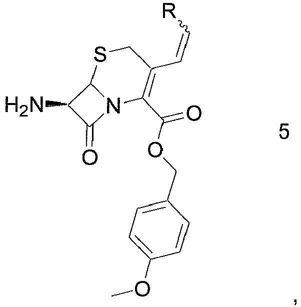 preferably the compound of Formula 5' 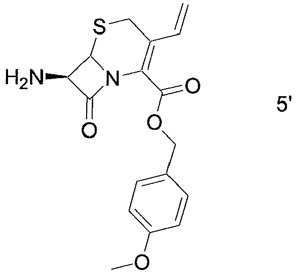 and the compound of Formula 4, optionally isolating the compound of Formula 5/5' from the reaction mixture, and
      (d) subjecting the compound of Formula 5/5' of step (c) to an enzymatic ester hydrolysis with p-nitrobenzyl esterase in order to provide the compound of Formula 6/6' and the compound of Formula 3; and
   (C) (synthesis sequence: Compound 1 -> Compound 2 + Compound 5 -> Compound 6) performing sequences A and B as a one-pot reaction by contacting the compound of Formula 1/1' of step (i) with *p*-nitrobenzyl esterase and pencillin G amidase; and
(iii) obtaining the compound of Formula 6/6'.

The compound of Formula 1/1' can be prepared according to methods known in the art, for example by applying a Wittig reaction as described in US 6,417,351 B1.

In one embodiment, the p-nitrobenzyl esterase and/or the pencillin G amidase can be immobilized and used as such. Immobilization techniques are known to a person skilled in the art. Useful solid supports include, e.g., polymer matrices such as calcium alginate, polyacrylamide, Eupergit^{®}, polymethacrylate and other polymeric materials, as well as inorganic matrices, such as Celite^{®}. Immobilization techniques are advantageous because the enzyme and the product can be easily separated. Additionally, the immobilized enzyme may be recycled and reused rendering the process more economic. Other techniques such as cross-linked enzyme aggregates (CLEAs) or cross-linked enzyme crystals (CLECs) are also applicable in the present invention.

Penicillin G Amidase was used from commercial suppliers (Iris Biotech GmbH, Epobond-PGA, Immobilised Penicillin G Amidase from *E*. *coli* on Epoxy Acrylic Polymer, 150 - 300 pm, activity > 140 U/g (wet), order number LS-1218.0100). This specific activity U is defined as the amount of enzyme which catalysis the hydrolysis of 1 µmol penicillin G in 20 mM phosphate buffer at pH 8 and 28°C per minute.

The above process includes the optional isolation of reaction products, which isolation / purification can be performed by applying techniques known in the art. Step (iii) comprises obtaining the compound of Formula 6 and may be performed by applying isolation and purification techniques known in the art. The enzyme can be removed either by filtration in case of immobilized enzyme or by other methods in case of soluble enzymes used, e.g. treatment with activated carbon, ultrafiltration etc. Compounds 3 and 4 can be extracted with organic solvents immiscible with water like ethyl acetate, MIBK (methyl isobutyl ketone) or others during the precipitation step of compound 6/6'. The crystallization of compound 6' can be done at a pH around 3. The precipitated product is isolated either by filtration or centrifugation, washed with water and optionally with an organic solvent and dried.

If the above process is performed without isolating intermediate products 2/2' or 5/5', the subsequent enzymatic reaction can be performed in the same reaction mixture or the reaction conditions can be modified to be more suitable for the subsequent enzymatic reaction, for example by modifying the pH value.

In step C of the above process, performing sequences A and B as a one-pot reaction by contacting the compound of Formula 1/1' of step (i) with *p*-nitrobenzyl esterase and pencillin G amidase can be performed by adding both enzymes to Compound 1/1', or vice versa, at the same time or by adding one enzyme after the other while there is still (a certain amount of) Compound 1/1' being present in the reaction mixture.

The p-nitrobenzyl esterase can be one of (i) to (iii):
(i) it can have at least 60 % sequence identity with SEQ ID NO: 1, over the whole length of SEQ ID NO: 1,
(ii) is a partial sequence of the enzyme having SEQ ID NO: 1, preferably the partial sequence has at least 400 amino acid residues; or
(iii) is derived from a partial sequence of the enzyme having SEQ ID NO: 1, wherein the fragment has less than 14 amino acid substitutions compared with the fragment of SEQ ID NO: 1, preferably the partial sequence has at least 400 amino acid residues, and
wherein the *p*-nitrobenzyl esterases defined in (i), (ii) and (iii) have at least 60% *p*-nitrobenzyl esterase activity compared with the enzyme having SEQ ID NO: 1 as determined in an activity assay with *p*-nitrophenylacetate as follows:
*p*-nitrophenylacetate (10 µL, 250 mM in DMSO) is mixed with sodium phosphate buffer (980 µL, 50 mM, pH 7), the reaction is performed at 30°C and is started by the addition of enzyme solution (10 µL with 8 - 15 mg/mL total protein) and monitored at 405 nm for 5 min.

The activity is calculated as follows: The absorption increase per minute is converted using the molar extinction coefficient for *p*-nitrophenyl to mol/min, which is converted to U/mL with the definition: 1 U is the enzyme amount which cleaves 1 µmol *p*-nitrophenylacetate at 30°C at pH 7 per minute. Any given activity values (U or IU), unless not otherwise defined, within this document refer to this definition of enzyme activity.

The calculation of percentages of identity can be performed with the help of well-known alignment algorithms and optionally computer programs using these algorithms. The identities can be calculated by aligning sequences with the freeware program ClustIX (Version 1.83) with default parameters and subsequent counting of identical residues by hand. Percentage identity (PID) can then be calculated by dividing the number of identities by the (entire) length of the shortest sequence. Default settings for, e.g., pairwise alignment (slow-accurate) are: gap opening parameter: 10.00; gap extension parameter 0.10; Protein weight matrix: Gonnet 250; DNA weight matrix IUB. Accordingly, as used herein, a nucleotide or amino acid sequence is said to have "X % sequence identity" to a given sequence if an alignment with the freeware program ClustaIX (Version 1.83) with default parameters, a subsequent determination of identical residues, such as by counting by hand, and a subsequent calculation of the percentage identity (PID) by dividing the number of identities by the (entire) length of the shortest sequence gives "X % sequence identity".

The *p*-nitrobenzyl esterase can be obtained from a wild type *Bacillus,* preferably from *Bacillus subtilis,* or is derived from said *p*-nitrobenzyl esterase by modification of the amino acid sequence, preferably by substitution of single amino acids, wherein said modified enzyme has at least 60% *p*-nitrobenzyl esterase activity compared with the enzyme having an amino acid sequence defined by SEQ ID NO: 1 as determined in an activity assay with *p*-nitrophenylacetate as defined above.

The *p*-nitrobenzyl esterase can be used in isolated form or is used as cell free extract or immobilized, preferably as cell free extract of *E*. *coli.*

Penicillin G amidase can be used in isolated form or is used as cell free extract or immobilized, preferably in immobilized form. Immobilization techniques are known to a person skilled in the art. Useful solid supports include, e.g., polymer matrices such as calcium alginate, polyacrylamide, Eupergit^{®}, and other polymeric materials, as well as inorganic matrices, such as Celite^{®}. Immobilization techniques are advantageous because the enzyme and the product can be easily separated. Additionally, the immobilized enzyme may be recycled and reused rendering the process more economic. Other techniques such as cross-linked enzyme aggregates (CLEAs) or cross-linked enzyme crystals (CLECs) are also applicable in the present invention.

Penicillin G Amidase can also be used from commercial suppliers as done in this disclosure (Iris Biotech GmbH, Epobond-PGA, Immobilised Penicillin G Amidase from *E*. *coli* on Epoxy Acrylic Polymer, 150 - 300 µm, activity > 140 U/g (wet), order number LS-1218.0100). This specific activity U is defined as the amount of enzyme which catalysis the hydrolysis of 1 µmol penicillin G in 20 mM phosphate buffer at pH 8 and 28°C per minute.

Preparation of the p-nitrobenzyl esterase, for example in purified form, is described in Zock J. et al. (1994) Gene 151 37 - 43 and EP 0 549 264 A1. The term "purified form" means that the enzyme has a ten-fold higher specific activity than *Bacillus* cell extracts. The *p*-nitrobenzyl esterase can for example be used in purified form or as cell free extract as described in the experimental part herein.

The wild type *Bacillus* can be selected from the group consisting of: *Bacillus subtilis* NRRL B558, *Bacillus circulons* ATCC 966, *Bacillus cereus* NRRL B569, *Bacillus lichenformis* ATCC 7072, *Bacillus cereus* ATCC 9634, *Bacillus subtilis* NRRL 1471, *Bacillus subtilis* NRRL B-8097, and *Bacillus subtilis* ATCC 6633, also mentioned in EP 0 549 264 A1 and US 3,972,774.

The *p*-nitrobenzyl esterase can be obtained from the expression system *E*. *coli* BL21 (DE3) Gold/pK470/pNBE wt or BL21(DE3) Gold/pK470/pNBE 5-6C8. Synthetic genes coding for pNBE wt and pNBE 5-6C8 were ordered from Integrated DNA Technologies, BVBA, Interleuvenlaan 12A, B-3001 Leuven, Belgium and cloned by standard techniques in the expression vector pK470. The expression plasmids were transformed into *E*. *coli* BL21 to obtain the expression strains.

A skilled person can choose suitable amounts of enzyme per substrate as well as concentrations thereof in the solvent/buffer system when performing the present invention. For example, 20-100 IU p-nitrobenzyl esterase per mg of Compound 1/1' can be used for synthesis alternative (ii)(A)(a). For example, 1 -100 IU penicillin G amidase per mg of Compound 1/1' can be used for synthesis alternative (ii)(B)(c). For example, 20-100 IU penicillin G amidase (e.g. the commercially available penicillin G amidase as mentioned herein) per mg of Compound 2/2' can be used for synthesis alternative (ii)(A)(b). For example, 20-100 IU *p*-nitrobenzyl esterase per mg of Compound 5/5' can be used for synthesis alternative (ii)(B)(d).

Enzyme preparations that can for example be used are: *p*-nitrobenzyl esterase in 0.5 M imidazole, 0.5 M NaCl and 20 mM NaPi, pH 7.40, having 2063 IU/mL; or *p*-nitrobenzyl esterase in 50 mM KPi, pH 7.50, having 4612 IU/mL.

The *p*-nitrobenzyl esterase can have an amino acid sequence according to SEQ ID NO: 1 or an amino acid sequence derived from SEQ ID NO: 1, i.e. a mutant thereof, with one or more substitutions selected from the group consisting of: isoleucine to valine at position 60; serine to glycine at position 94; asparagine to serine at position 96; leucine to methionine at position 144; lysine to arginine at position 267; phenylalanine to leucine at position 271; proline to serine at position 317; histidine to arginine at position 322; leucine to valine at position 334; leucine to serine at position 334; alanine to valine at position 343; methionine to valine at position 358; and tyrosine to phenylalanine at position 370.

A preferred mutant of p-nitrobenzyl esterase is the 5-6C8 enzyme having an amino acid sequence derived from SEQ ID NO: 1 by the following modifications: isoleucine to valine at position 60; leucine to methionine at position 144; proline to serine at position 317; histidine to arginine at position 322; leucine to serine at position 334; methionine to valine at position 358; and tyrosine to phenylalanine at position 370.

Mutants of *p*-nitrobenzyl esterases and processes for preparing same by modification of *p-*nitrobenzyl esterases from *Bacillus subtilis* are described in US 5,945,325. The 5-6C8 mutant is described in Spiller et al. (Proc Natl Acad Sci USA. 1999 October 26; 96(22): 12305-12310). The above mutants of p-nitrobenzyl esterases have improved stability and/or ester hydrolysis activity in organic media (see Moore, J. C. and Arnold, F. H. (1996), Nat. Biotech 14:458-467 and Moore, J. C. et al. (1997) J. Mol. Biol. 272, 336-347 and US 5,741,691).

The enzymatic ester hydrolysis in step (ii)(A)(a), (ii)(B)(d) and (ii)(C) can be performed in a buffered system at a pH in the range of 6-8, preferably 6.5-7.5, wherein the buffered system contains an aqueous buffer and an organic solvent or a mixture of organic solvents, wherein the organic solvent or mixture of organic solvents used in step (ii)(A)(a) is preferably chosen to provide a higher solubility of the compound of Formula 1/1' as compared with the same amount of water at 25°C and wherein the organic solvent or mixture of organic solvents used in step (ii)(B)(d) is preferably chosen to provide a higher solubility of the compound of Formula 5/5' as compared with the same amount of water at 25°C. The total amount of organic solvents in the reaction mixture is preferably limited to avoid a significantly reduced activity of the enzymes used. For example, it can be limited to 30%, 20% or 15% by volume based on the total volume of the buffered system.

The enzymatic hydrolysis in step (ii)(A)(b) and (ii)(B)(c) is performed in a buffered system at a pH in the range of 6-8, preferably 6.5-7.5, wherein the buffered system contains an aqueous buffer and an organic solvent or a mixture of organic solvents, wherein the organic solvent or mixture of organic solvents used in step (ii)(A)(b) is preferably chosen to provide a higher solubility of the compound of Formula 2/2' as compared with the same amount of water at 25°C and wherein the organic solvent or mixture of organic solvents used in step (ii)(B)(c) is preferably chosen to provide a higher solubility of the compound of Formula 1/1' as compared with the same amount of water at 25°C. The total amount of organic solvents in the reaction mixture is preferably limited to avoid a significantly reduced activity of the enzymes used. For example, it can be limited to 30%, 20% or 15% by volume based on the total volume of the buffered system.

The buffered system can comprise one or more aqueous buffers known in the art, for example buffers selected from the group consisting of sodium phosphate buffer, potassium phosphate buffer, sodium acetate buffer, 4-(2-hydroxyethyl)-1-piperazineethanesulfonic acid, tris(hydroxymethyl)aminomethane, preferably a sodium phosphate buffer is used.

The organic solvent(s) can be selected from the group consisting of dimethyl sulfoxide, ethanol, methanol, ethyl acetate, isoamyl alcohol, dichloromethane, acetonitrile, iso-propanol, chloroform, dimethyl formamide, tetrahydrofurane and cyclohexane, preferably dimethyl sulfoxide is used as the organic solvent.

The buffer, preferably sodium phosphate buffer, can have a molarity in the range of from 10 to 50 mM, preferably 10-30 mM, further preferred about 10 mM, wherein the buffer is preferably used in an amount of 60-95 % by volume based on the total volume of the buffered system and the organic solvent(s), preferably dimethyl sulfoxide, is/are used in an amount of 5-40% by volume based on the total volume of the buffered system. The buffered system preferably consists of buffer and organic solvent(s).

The enzymatic ester hydrolysis by using *p*-nitrobenzyl esterases can be performed in a buffered system at a pH in the range of 6-8, preferably 6.5-7.5.

The *p*-nitrobenzyl esterase can have an amino acid sequence according to SEQ ID NO: 1 and the buffer, preferably sodium phosphate buffer, can have a molarity in the range of from 10 and 50 mM, preferably 10 mM, and can be used in an amount of 70-98 % by volume based on the total volume of the buffered system and the organic solvent, preferably dimethyl sulfoxide, can be used in an amount of 2-30% by volume based on the total volume of the buffered system. The p-nitrobenzyl esterase can also for example be the 5-6C8 enzyme as defined herein and the buffer, preferably sodium phosphate buffer, can have a molarity in the range of from 10 and 50 mM, preferably 40 mM, and is used in an amount of 70-98 % by volume based on the total volume of the buffered system and the organic solvent, preferably dimethyl sulfoxide, is used in an amount of 2-30% by volume based on the total volume of the buffered system.

It is particularly preferred to use 8-12%, preferably about 10%, of dimethyl sulfoxide (as the sole organic solvent or other solvents in amounts of less than 5%) by volume based on the total volume of the buffered system when using the wild type form of p-nitrobenzyl esterase.

It is particularly preferred to use 6-10%, preferably about 6-8%, of dimethyl sulfoxide (as the sole organic solvent or other solvents in amounts of less than 5%) by volume based on the total volume of the buffered system when using the 5-6C8 mutant enzyme.

Preferably, the salt concentration in the reaction mixture(s) of the process of the invention is less than 50 mM, or less than 20 mM, preferably about 10 mM.

The enzymatic ester hydrolysis can be performed at a temperature in the range of from 25 to 45°C, wherein the enzymatic ester hydrolysis with the p-nitrobenzyl esterase having an amino acid sequence according to SEQ ID NO: 1 is preferably performed at a temperature in the range of from 25 to 35°C, and the enzymatic ester hydrolysis with the enzyme 5-6C8 as defined herein is preferably performed at a temperature in the range of from 33 to 43°C. Higher temperature increased conversion with the mutant.

The amide bond cleavage in alternatives (A) and (B) is performed by enzymatic amide bond cleavage with pencillin G amidase ([EC 3.5.1.11]). The systematic name of this enzyme class is penicillin amidohydrolase. Other names include penicillin acylase, benzylpenicillin acylase, novozym 217, semacylase, alpha-acylamino-beta-lactam acylhydrolase, and ampicillin acylase.

The pencillin G amidase was used from commercial supplier Iris Biotech GmbH, Epobond-PGA, Immobilised Penicillin G Amidase from *E*. *coli* on Epoxy Acrylic Polymer, 150-300pm, Activity >140 U/g(wet), order number LS-1218.0100. This specific activity U is defined as the amount of enzyme which catalysis the hydrolysis of 1 µmol penicillin G in 20 mM phosphate buffer at pH 8 and 28°C per minute. A person skilled in the art is also able to produce penicillin G amidase having a desired sequence.

The enzymatic amide bond cleavage with pencillin G amidase can be performed in a buffer, preferably potassium phosphate buffer, preferably having a concentration of from 50 to 200 mM, at a pH in the range of from 7 to 8, at a temperature in the range of from 20 to 40°C.

The invention also refers to a process for the preparation of the compound of Formula 2/2' comprising steps (i) (providing a compound of Formula 1/1') and (ii)(A)(a) as defined herein, and a step of obtaining the compound of Formula 2/2'.

The invention also refers to a process for the preparation of 7-amino-3-alkenylcephem-4-carboxylic acid of Formula 6, preferably of Formula 6', comprising steps (I) to (III):
(I) providing a compound of Formula 5/5';
(II) subjecting the compound of Formula 5/5' to a process as defined in step (ii)(B)(d) herein; and
(III) obtaining the compound of Formula 6/6'.

The invention also refers to a process for the preparation of the compound of Formula 5, preferably of Formula 5', comprising step (i) (providing a compound of Formula 1) and (ii)B(c) as defined herein and an additional step of obtaining the compound of Formula 5/5'.

The invention also refers to the use of a *p*-nitrobenzyl esterase as defined herein for the preparation of the compound of Formula 2/2' or 6/6'.

The invention also refers to the use of *p*-nitrobenzyl esterase as defined herein and pencillin G amidase as defined herein for the preparation of the compound of Formula 6/6'.

The following examples describe the present invention in detail, but are not to be construed to be in any way limiting for the present invention.

### Example 1 - Preparation of p-nitrobenzyl esterase

An overnight culture (30 mL 2xTY (trypton yeast extract) medium, 40 ug/mL kanamycin) inoculated with a single colony of *E*. *coli* BL21(DE3) Gold/pK470/pNBE wt or BL21(DE3) Gold/pK470/pNBE 5-6C8, obtained by cloning synthetic genes coding for pNBE wt and pNBE 5-6C8 by standard techniques in the expression vector pK470 and transformation of the expression plasmids into *E*. *coli BL21,* was incubated at 30°C and 150 rpm overnight.

A preculture (50 mL 2xTY medium, 40 µg/mL kanamycin) was inoculated to OD₆₀₀ = 0.1 and incubated at 30°C and 120 rpm until OD₆₀₀= 1.

Then, the main culture (300 mL 2xTY medium, 40 µg/mL kanamycin) was inoculated to OD₆₀₀ = 0.1 and incubated at 30°C und 120 rpm until OD₆₀₀ = 0.7 - 0.9. The expression was induced by the addition of IPTG (Isopropyl β-D-1-thiogalactopyranoside) [100 µM] and the cultures were further incubated at 28°C and 120 rpm overnight. The cells were harvested by centrifugation at 4000 rpm and 4°C for 20 min.

The cell pellets were then disrupted in NaPi (sodium phosphate) buffer (50 mM, pH 7, 10 mL/1 g wet cells) via ultrasonification for 5 min under ice bath cooling. The cell debris was centrifuged of for 20 min at 20000 rpm and 4°C to yield the cell free extract.

### Example 2 - Source of penicilin G amidase

Iris Biotech GmbH, Epobond-PGA, Immobilised Penicillin G Amidase from *E*. *coli* on Epoxy Acrylic Polymer, 150-300µm, Activity >140 U/g (wet), order number LS-1218.0100. This specific activity U is defined as the amount of enzyme which catalysis the hydrolysis of 1 µmol penicillin G in 20 mM phosphate buffer at pH 8 and 28°C per minute.

### Example 3 - Activity assay with p-Nitrophenylacetate

p-nitrophenylacetate (10 µL, 250 mM in DMSO) was mixed with NaPi buffer (980 µL, 50 mM, pH 7). The reaction was started by the addition of enzyme solution (10 µL with 8 - 15 mg/mL total protein) and monitored 405 nm for 5 min. The reaction is performed at 30°C with a Beckman-Coulter DU800 Spectrophotometer with Temperature Controller. The activity of the enzyme is obtained from the experimental results by using the approach as described in more detail above. Result: pNBE wt: 7656 IU/mL, 8.5 mg/mL pNBE 5-6C8: 4300 IU/mL, 12 mg/mL

### Example 4 - Analytics

### HPLC:

Column: Chromolith Performance RP-18e 100-4.6
Flow: 1 mL/min; Temperature: 25°C; Wavelength: 254 nm
Eluent A: 0.05% acetic acid
Eluent B: acetonitrile
Gradient: 0 min/20 %B - 7 min/80 %B - 7.05 min/100 %B - 9 min/100 %B - 10 min/20 %B

### References:

Compound 1': 8.6 min; Compound 2': 4.6 min; Anisalcohol: 4.1 min; Compound 5': 1.4 min; Compound 6': 1.53 min.

### Example 5 - System for determining suitable conditions for ester hydrolysis

The p-nitrobenzyl esterase from *Bacillus subtilis* and its mutant 5-6C8 showed a cleavage/hydrolysis activity on the compound of Formula 1' yielding the compound of Formula 2' as the sole product according to HPLC (high-performance liquid chromatography) measurements. The first test reactions (see Figure 2) were performed in NaPi (sodium phosphate) buffer (50mM, pH 7) using 30% (v/v) DMSO (dimethyl sulfoxide) as cosolvent for the compound of Formula 1' which is not soluble in aqueous buffer solutions. With this set up the wild type enzyme showed only 12% conversion, the mutant reached 34%.

### Example 6 - Determining suitable amounts of organic solvent

For all reactions 430 IU of enzyme were used.

G-Vinyl-E stock (1 M): 0.434 g GVinylE was dissolved in 1 mL DMSO.

**Table 1: Pipetting scheme for reactions (µL)**

| **pNBE wt** | **DMSO (v/v%)** | | | | | | |
|---|---|---|---|---|---|---|---|
| **Compound** | **30** | **20** | **10** | **8** | **6** | **4** | **2** |
| Buffer | 295 | 345 | 395 | 405 | 415 | 425 | 435 |
| DMSO | 140 | 90 | 40 | 30 | 20 | 10 | - |
| GVinylE | 10 | 10 | 10 | 10 | 10 | 10 | 10 |
| Lysate | 55 | 55 | 55 | 55 | 55 | 55 | 55 |
| | | | | | | | |

| **pNBE 5-6C8** | **DMSO (v/v%)** | | | | | | |
|---|---|---|---|---|---|---|---|
| **Compound** | **30** | **20** | **10** | **8** | **6** | **4** | **2** |
| Buffer | 250 | 300 | 350 | 360 | 370 | 380 | 390 |
| DMSO | 140 | 90 | 40 | 30 | 20 | 10 | - |
| GVinylE | 10 | 10 | 10 | 10 | 10 | 10 | 10 |
| Lysate | 100 | 100 | 100 | 100 | 100 | 100 | 100 |

All reactions were incubated at 30°C and 700 rpm on a thermomixer overnight and stopped by the addition of acetonitrile (500 µL). One further sample with 30 % (v/v) DMSO of the wt (wild type) and the mutant (5-6C8) were incubated at 37°C.

The obtained conversion levels for the reduction of DMSO in the samples are summarized in the table below. The starting conditions for these optimization reactions were 30 % (v/v) DSMO in the reaction mixture and incubation at 30°C. The last entry has been performed at 37°C.

**Table 2.**

| **DMSO** | **Conversion (%)** | |
|---|---|---|
| **% (v/v)** | **wt** | **5-6C8** |
| 2 | 9,78 | 18,53 |
| 4 | 25,89 | 23,86 |
| 6 | 30,38 | 38,98 |
| 8 | 33,04 | 42,26 |
| 10 | 53,08 | 30,16 |
| 20 | 42,37 | 39,93 |
| 30 | 12,43 | 34,11 |
| 30 | 5,20 | 48,30 |

For both the wt and the mutant the conversion is increased with the reduction of DMSO present in the reaction solution. For the wt enzyme a 4.3 fold increase if compared to 30% (v/v) DMSO is achieved by reducing DMSO to 10% (v/v). For the mutant a 1.2 fold increase can be detected by reducing DMSO to 8% (v/v) from 30% (v/v) DMSO. Furthermore the conversion with the mutant is also better with higher temperature, which is not observed for the wt enzyme.

### Example 7 - Determining suitable buffer systems

For all reactions 430 IU of enzyme and 10% (v/v) DMSO were used.

Stock preparation of the Compound 1'(1 M): 0.434 g of the compound of Formula 1' was dissolved in 1 mL DMSO.

**Table 3. Pipetting scheme (µL) List of buffers used**

| **Compound** | **wt** | **5-6C8** | | **Nr** | **Salt** | **Conc. (mM)** | **pH** |
|---|---|---|---|---|---|---|---|
| Buffer | 395 | 350 | | 1 | NaPi | 40 | 7 |
| DMSO | 40 | 40 | | 2 | NaPi | 30 | 7 |
| Compound 1' | 10 | 10 | | 3 | NaPi | 20 | 7 |
| Lysate | 55 | 100 | | 4 | NaPi | 10 | 7 |
| | | | | 5 | NaPi | 50 | 8 |
| | | | | 6 | KPi | 50 | 7 |
| | | | | 7 | HEPES | 50 | 7 |
| | | | | 8 | Tris | 50 | 7 |
| | | | | 9 | Tris | 50 | 9 |
| | | | | 10 | NaOAc | 50 | 4,5 |
| | | | | 11 | KPi | 50 | 6 |
| | | | | 12 | NaPi | 50 | 7 |

The abbreviations in the above table and used herein are as follows: NaPi (sodium phosphate), KPi (potassium phosphate), Tris (tris(hydroxymethyl)aminomethane), HEPES (4-(2-hydroxyethyl)-1-piperazineethanesulfonic acid), NaOAc (sodium acetate), wt (wild type of *p-*nitrobenzyl esterase of *Bacillus subtilis*), 5-6C8 (mutant of wt).

All wt samples were incubated at 30°C, all 5-6C8 samples were incubated at 37°C overnight and stopped by the addition of acetonitrile (500 µL).

The results for the buffer variation are summarized in the table below. The reduction of NaPi ions in the buffer does influence the conversion in a positive way; it is possible to reduce the concentration to 10 mM buffer salts. Furthermore also other buffer systems in the range of pH 7 are possible for the reaction. Raising the pH value is not favorable due to the instability of the substrate at a pH over 8.

**Table 4.**

| **Buffer** | | | | **Conversion (%)** | |
|---|---|---|---|---|---|
| **Nr** | **Salt** | **Conc. (mM)** | **pH** | **wt (30°C)** | **5-6C8 (37°C)** |
| 1 | NaPi | 40 | 7 | 61,38 | 91,05 |
| 2 | NaPi | 30 | 7 | 53,70 | 74,64 |
| 3 | NaPi | 20 | 7 | 55,85 | 89,26 |
| 4 | NaPi | 10 | 7 | 72,24 | 77,02 |
| 5 | NaPi | 50 | 8 | 52,37 | - |
| 6 | KPi | 50 | 7 | 41,46 | 81,06 |
| 7 | HEPES | 50 | 7 | 46,66 | 72,80 |
| 8 | Tris | 50 | 7 | 60,59 | 62,55 |
| 9 | Tris | 50 | 9 | - | - |
| 10 | NaOAc | 50 | 4,5 | - | - |
| 11 | KPi | 50 | 6 | 28,91 | - |
| 12 | NaPi | 50 | 7 | 35,21 | 75,98 |

### Example 8 - Determining suitable organic solvents/cosolvents

Compound 1' (4.6 mg) was added to 50 µL of the below organic solvents. Then, the buffer and lysate was added. All wt samples were incubated at 30°C, all 5-6C8 samples were incubated at 37°C overnight.

**Table 5. Pipetting scheme**

| **Compound** | **wt** | **5-6C8** |
|---|---|---|
| Buffer (µL) | 395 | 350 |
| Org. Solvent (µL) | 50 | 50 |
| Compound 1'(mg) | 4.6 | 4.6 |
| Lysate (µL) | 55 | 100 |

All wt samples were incubated at 30°C, all 5-6C8 samples were incubated at 37°C overnight.

None of the tested organic solvents, i.e. ethanol, methanol, ethyl acetate, isoamyl alcohol, dichloromethane, acetonitrile, iso-propanol, chloroform, dimethyl formamide, tetrahydrofurane and cyclohexane, resulted in a higher conversion than 10% DMSO.

### Example 9 - Preparation of Compound 5'(pMB-VICA) from Compound 1' (G-Vinyl-E)

Compound 1' (5.0 mg/mL) was contacted with immobilised penicilin G amidase (Iris Biotech GmbH, Epobond-PGA, Immobilised Penicillin G Amidase from *E*. *coli* on Epoxy Acrylic Polymer, 150-300µm, Activity >140 U/g (wet), order number LS-1218.0100; This specific activity U is defined as the amount of enzyme which catalysis the hydrolysis of 1 µmol penicillin G in 20 mM phosphate buffer at pH 8 and 28°C per minute.) (10.0 U/mL reaction volume), in 5 vol% DMSO and 100 mM KPi at pH 7.50 at room temperature.
Conversion to 5' after 68h: 8%

### Example 10 - Preparation of Compound 6'(VICA) from Compound 2' (G-VICA)

5 mL scale: Compound 2' (1.0 mmol) was contacted with 700 mg immobilised penicilin G amidase (Iris Biotech GmbH, Epobond-PGA, Immobilised Penicillin G Amidase from *E*. *coli* on Epoxy Acrylic Polymer, 150-300pm, Activity >140 U/g (wet), order number LS-1218.0100; This specific activity U is defined as the amount of enzyme which catalysis the hydrolysis of 1 µmol penicillin G in 20 mM phosphate buffer at pH 8 and 28°C per minute.) in 100 mM KPi at pH 7.50 at room temperature; pH adjustment was performed with 10 M NaOH; Full conversion was achieved after 30 h reaction time.

**Table 6.**

| **Entry** | **Compound 2' [mg]** | **purity [%]** | **conversion to Compound 6'** | **isolated product [%]*)** | **purity at 290 nm [%]** |
|---|---|---|---|---|---|
| 2 | 336 | 87 | 100% | 28 | >99.5 |
| 4 | 311 | 96 | 100% | 49 | >99 |

| | | | | | |
|---|---|---|---|---|---|
| *) precipitation at pH 4.0 | | | | | |

### Example 11 - Preparation of Compound 6'(VICA) from Compound 5' (pMB-VICA)

10 µL of 1 M pMB-VICA in DMSO were added to 140 µL DMSO and 250 µL buffer. Reaction was started by addition of 100 µL 5-6C8 lysate (430 IU). Reaction was performed at 37°C for 24 hours on a thermomixer with 700 rpm.

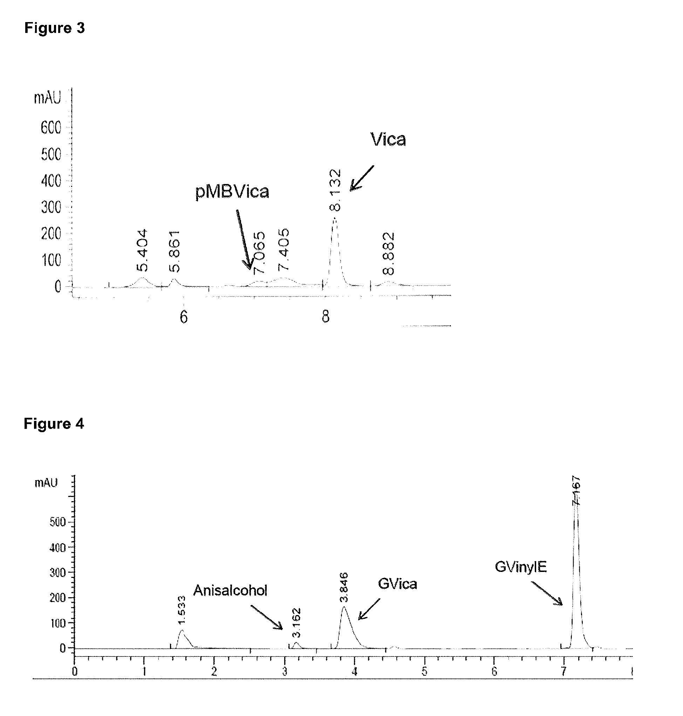

The reaction mixture was analyzed by HPLC, see Figure 3

### Example 12 - Preparation of Compound 2'(G-VICA) from Compound 1' (G-Vinyl-E)

GVinylE (0.43 g, 1 mmol) were dissolved DMSO (15 mL) then NaPi buffer (25 mL, 50 mM, pH 7) was added. The reaction was started by the addition of 10 mL cell free extract (44.500 IU). The reaction was performed on a rotary shaker at 30°C and 120 rpm 24h.

The reaction mixture was analyzed by HPLC, see Figure 4

### Example 13 - One-pot reaction: Preparation of Compound 6'(VICA) from Compound 1' (G-Vinyl-E)

5 mg of Compound 1' in 100 mM K₂HPO₄ and 5% (v/v) DMSO at pH 7.50 was contacted with 231 IU/mL of wt (wild type) PNB esterase or 260 IU/mL PNB esterase 5-6C8 mutant and PGA (3.0 mg/mL); Conversion after 68h was analyzed. No Compounds 2' and 5' were detected in the reaction mixture.

**Table 7.**

| esterase | conversion to 6' |
|---|---|
| wt | 89% |
| 5-6C8 mutant | 97% |

### Cited literature

EP 0 549 264 A1;
US 3,972,774;
US 5,741,691;
US 5,945,325 B1;
US 6,417,351 B1;
US 6,420,554;
Chao et al., "An Improved and Scaleable Preparation of 7-Amino-3-vinylcephem-4-carboxylic acid", Organic Process Research & Development 2009, 13, 924-927;
Hu et al., J. China Pharm. Univ., 1993, 24(4):246-247; and
B. Spiller et al., "A structural view of evolutionary divergence", Proc Natl Acad Sci U S A. 1999 October 26; 96(22): 12305-12310.
Moore, J. C. and Arnold, F. H. (1996), "Directed evolution of a para-nitrobenzyl esterase for aqueous-organic solvents", Nat. Biotech 14:458-467
Moore, J. C. et al. (1997), "Strategies for the in vitro evolution of protein function: Enzyme evolution by random recombination of improved sequences", J. Mol. Biol. 272, 336-347
Zock J. et al. (1994) Gene, "The Bacillus subtilis pnbA gene encoding p-nitrobenzyl esterase: cloning, sequence and high-level expression in Escherichia coli", 151: 37-43

## Claims

1. Process for the preparation of 7-amino-3-alkenylcephem-4-carboxylic acid of Formula 6 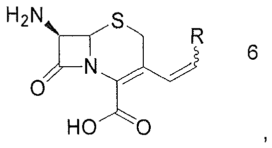 wherein R = H or methyl, preferably R = H according to Formula 6' 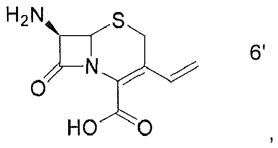 comprising steps (i) to (iii):
(i) providing a compound of Formula 1 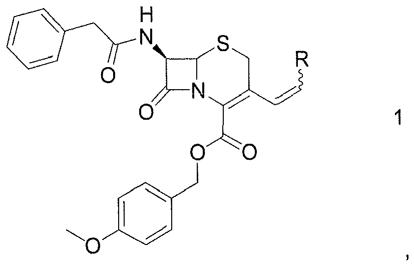 wherein R is as defined above;
(ii) performing one of alternatives (A), (B) and (C):
(A)
(a) subjecting the compound of Formula 1 of step (i) to an enzymatic ester hydrolysis by using *p*-nitrobenzyl esterase in order to provide the compound of Formula 2 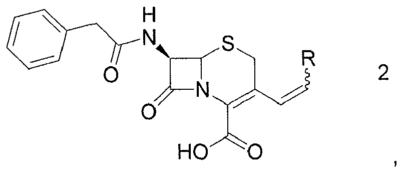 wherein R is as defined above, and the compound of Formula 3 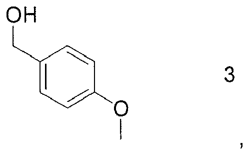 and
(b) subjecting the compound of Formula 2 of step (a) to an amide bond cleavage by using pencillin G amidase in order to provide the compound of Formula 6 and the compound of Formula 4 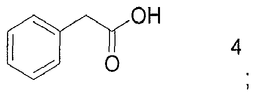
(B)
(c) subjecting the compound of Formula 1 of step (i) to an amide bond cleavage by using pencillin G amidase in order to provide the compound of Formula 5 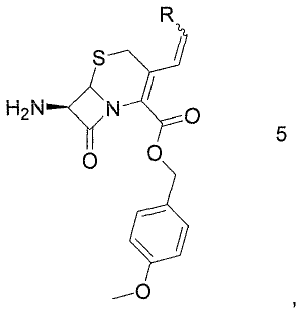 wherein R is as defined above;
and the compound of Formula 4, and
(d) subjecting the compound of Formula 5 of step (c) to an enzymatic ester hydrolysis with *p*-nitrobenzyl esterase in order to provide the compound of Formula 6 and the compound of Formula 3; and
(C)
performing sequences A and B as a one-pot reaction by contacting the compound of Formula 1 of step (i) with *p*-nitrobenzyl esterase and pencillin G amidase; and
(iii) obtaining the compound of Formula 6.

2. The process of claim 1, wherein the *p*-nitrobenzyl esterase
(i) has at least 60 % sequence identity with SEQ ID NO: 1,
(ii) is a partial sequence of the enzyme having SEQ ID NO: 1, preferably the partial sequence has at least 400 amino acid residues, or
(iii) is derived from a partial sequence of the enzyme having SEQ ID NO: 1, wherein the fragment has less than 14 amino acid substitutions compared with the fragment of SEQ ID NO: 1, preferably the partial sequence has at least 400 amino acid residues, and
wherein the *p*-nitrobenzyl esterases defined in (i), (ii) and (iii) have at least 60% *p-*nitrobenzyl esterase activity or at least 4000 IU/mL at 8 - 15 mg/mL protein compared with the enzyme having SEQ ID NO: 1 as determined in an activity assay with *p-*nitrophenylacetate as follows:
*p*-nitrophenylacetate (10 µL, 250 mM in DMSO) is mixed with sodium phosphate buffer (980 µL, 50 mM, pH 7), the reaction is performed at 30°C and is started by the addition of enzyme solution (10 µL with 8 - 15 mg/mL total protein) and monitored at 405 nm for 5 min.

3. The process of claim 1 or 2, wherein the *p*-nitrobenzyl esterase is obtained from a wild type *Bacillus,* preferably from *Bacillus subtilis,* or is derived from said *p-*nitrobenzyl esterase by modification of the amino acid sequence, preferably by substitution of single amino acids, wherein said modified enzyme has at least 60% *p-*nitrobenzyl esterase activity compared with the enzyme having an amino acid sequence defined by SEQ ID NO: 1 as determined in an activity assay with *p-*nitrophenylacetate as defined in claim 2.

4. The process of any of claims 1-3, wherein the p-nitrobenzyl esterase has an amino acid sequence according to SEQ ID NO: 1 or an amino acid sequence derived from SEQ ID NO: 1 with one or more substitutions selected from the group consisting of: isoleucine to valine at position 60; serine to glycine at position 94; asparagine to serine at position 96; leucine to methionine at position 144; lysine to arginine at position 267; phenylalanine to leucine at position 271; proline to serine at position 317; histidine to arginine at position 322; leucine to valine at position 334; leucine to serine at position 334; alanine to valine at position 343; methionine to valine at position 358; and tyrosine to phenylalanine at position 370.

5. The process of any of claims 1-4, wherein the *p*-nitrobenzyl esterase is the 5-6C8 enzyme having an amino acid sequence derived from SEQ ID NO: 1 by the following modifications: isoleucine to valine at position 60; leucine to methionine at position 144; proline to serine at position 317; histidine to arginine at position 322; leucine to serine at position 334; methionine to valine at position 358; and tyrosine to phenylalanine at position 370.

6. The process of any of claims 1-5, wherein the enzymatic ester hydrolysis in step (ii)(A)(a), (ii)(B)(d) and (ii)(C) is performed in a buffered system at a pH in the range of 6-8, preferably 6.5-7.5, wherein the buffered system contains an aqueous buffer and an organic solvent or a mixture of organic solvents, wherein the organic solvent or mixture of organic solvents used in step (ii)(A)(a) is preferably chosen to provide a higher solubility of the compound of Formula 1 as compared with the same amount of water at 25°C and wherein the organic solvent or mixture of organic solvents used in step (ii)(B)(d) is preferably chosen to provide a higher solubility of the compound of Formula 5 as compared with the same amount of water at 25°C.

7. The process of claim 6, wherein the buffered system comprises one or more aqueous buffers selected from the group consisting of sodium phosphate buffer, potassium phosphate buffer, sodium acetate buffer, 4-(2-hydroxyethyl)-1-piperazineethanesulfonic acid, tris(hydroxymethyl)aminomethane, preferably a sodium phosphate buffer is used.

8. The process of claim 6 or 7, wherein the organic solvent(s) is/are selected from the group consisting of dimethyl sulfoxide, ethanol, methanol, ethyl acetate, isoamyl alcohol, dichloromethane, acetonitrile, iso-propanol, chloroform, dimethyl formamide, tetrahydrofurane and cyclohexane, preferably dimethyl sulfoxide is used as the organic solvent.

9. The process of any of claims 6-8, wherein the buffer, preferably sodium phosphate buffer, has a molarity in the range of from 10 to 50 mM, preferably 10-30 mM, further preferred about 10 mM, wherein the buffer is preferably used in an amount of 60-95 % by volume based on the total volume of the buffered system and the organic solvent(s), preferably dimethyl sulfoxide, is/are used in an amount of 5-40% or 5-20%, preferably 8-12% or 6-10%, such as 8-10% or 8-14% of dimethyl sulfoxide as the sole organic solvent, by volume based on the total volume of the buffered system.

10. Process for the preparation of the compound of Formula 2 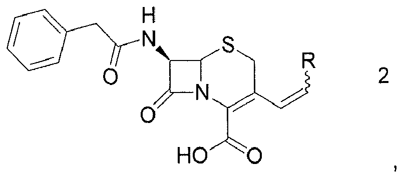 wherein R = H or methyl, wherein if R = H, the compound is defined by Formula 2' 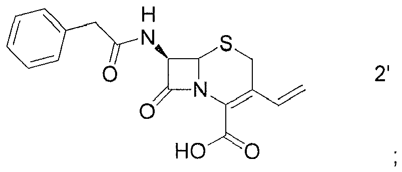 comprising steps (i) and (ii)(A)(a) as defined in any of the preceding claims, and a step of obtaining the compound of Formula 2.

11. Process for the preparation of 7-amino-3-alkenylcephem-4-carboxylic acid of Formula 6 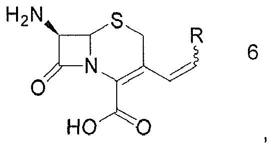 wherein R = H or methyl, preferably R = H according to Formula 6' 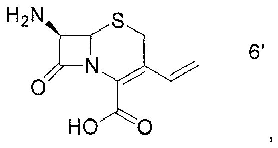 comprising steps (I) to (III):
(I) providing a compound of Formula 5 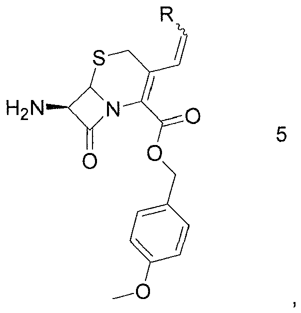 wherein R is as defined above;
(II) subjecting the compound of Formula 5 to a process as defined in step (ii)(B)(d) of any of claims 1-9; and
(III) obtaining the compound of Formula 6.

12. Process for the preparation of the compound of Formula 5 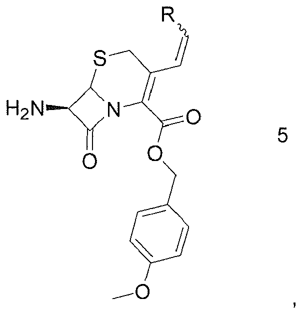 wherein R = H or methyl, preferably R = H according to Formula 5' 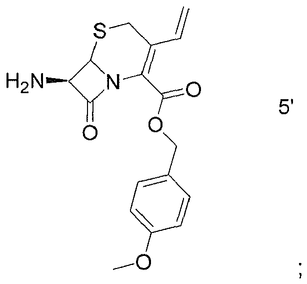 comprising step (i) and (ii)B(c) as defined in claim 1 and an additional step of obtaining the compound of Formula 5.

13. Use of a *p*-nitrobenzyl esterase as defined in any of claims 2-5 for the preparation of the compound of Formula 2 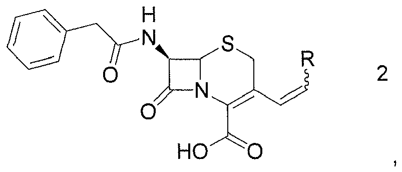 wherein R = H or methyl, preferably R = H;
or the compound of Formula 6 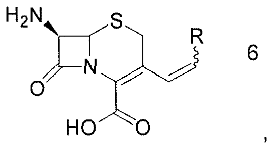 wherein, R = H or methyl, preferably R = H.

14. Use of *p*-nitrobenzyl esterase as defined in any of claims 2-5 and pencillin G amidase for the preparation of the compound of Formula 6 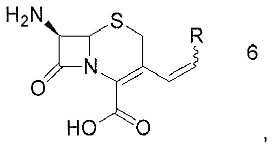 wherein, R = H or methyl, preferably R = H.
